# EUROPÄISCHE PATENTSCHRIFT

(11) **EP 1 682 467 B1**
(45) Veröffentlichungstag und Bekanntmachung des Hinweises auf die Patenterteilung: **10.08.2016**
(21) Anmeldenummer: 04791040.1
(22) Anmeldetag: 29.10.2004
(51) Int. Cl.: C07C 2/66, C11D 1/22

(54) **VERFAHREN ZUR HERSTELLUNG VON ALKYLARYLVERBINDUNGEN UND SULFONATEN DAVON**
METHOD FOR PRODUCING ALKYLARYL COMPOUNDS AND ALKYLARYL SULPHONATES
PROCEDE DE PRODUCTION D'ALKYLARYLES ET D'ALKYLARYLSULFONATES

(30) Priorität: 29.10.2003 DE 10350333
(43) Veröffentlichungstag der Anmeldung: 26.07.2006
(73) Patentinhaber: BASF SE, 67056 Ludwigshafen am Rhein (DE)
(72) Erfinder: BOTTKE, Nils, 68165 Mannheim (DE); BENFER, Regina, 67122 Altrip (DE); BOSCH, Marco, 68159 Mannheim (DE); NARBESHUBER, Thomas, 68165 Mannheim (DE); STEINBRENNER, Ulrich, 67435 Neustadt (DE); STEPHAN, Jürgen, 68163 Mannheim (DE)
(74) Vertreter: BASF IP Association
(86) Internationale Anmeldenummer: PCT/EP2004/012280
(87) Internationale Veröffentlichungsnummer: WO 2005/042448

(56) Entgegenhaltungen:
- WO-A-03/029172

## Beschreibung

Die vorliegende Erfindung betrifft Verfahren zur Herstellung von Alkylarylverbindungen und Alkylarylsulfonaten, nach diesen Verfahren erhältliche Alkylaryle und Alkylarylsulfonate, die Verwendung letzterer als Tenside, vorzugsweise in Wasch- und Reinigungsmitteln, und diese enthaltende Wasch- und Reinigungsmittel.

Alkylbenzolsulfonate (ABS) werden seit langer Zeit als Tenside in Wasch- und Reinigungsmitteln eingesetzt. Nachdem zunächst derartige Tenside auf Basis von Tetrapropylenbenzolsulfonat eingesetzt wurden, die jedoch schlecht biologisch abbaubar waren, wurden in der Folgezeit möglichst lineare Alkylbenzolsulfonate (LAS) hergestellt und verwendet. Lineare Alkylbenzolsulfonate weisen jedoch nicht in allen Anwendungsbereichen ausreichende Eigenschaftsprofile auf.

So wäre es zum Beispiel vorteilhaft, ihre Kaltwascheigenschaften oder ihre Eigenschaften in hartem Wasser zu verbessern. Ebenso wünschenswert ist die leichte Formulierbarkeit, gegeben durch die Viskosität der Sulfonate und deren Löslichkeit. Diese verbesserten Eigenschaften zeigen geringfügig verzweigte Verbindungen bzw. Mischungen von geringfügig verzweigten Verbindungen mit linearen Verbindungen, wobei man jedoch das richtige Maß an Verzweigung und/oder das richtige Maß an Mischung erzielen muss. Zu starke Verzweigungen benachteiligen die biologische Abbaubarkeit der Produkte. Zu lineare Produkte beeinflussen die Viskosität und die Löslichkeit der Sulfonate negativ.

Darüber hinaus spielt das Verhältnis von terminalen Phenylalkanen (2-Phenylalkane und 3-Phenylalkane) zu internen Phenylalkanen (4-, 5-, 6- etc. Phenylalkane) eine Rolle für die Produkteigenschaften. Ein 2-Phenylanteil von etwa 20 - 40 % und ein 2- und 3-Phenylanteil von etwa 40 - 60 % können hinsichtlich der Produktqualität (Löslichkeit, Viskosität, Wascheigenschaften, biologische Abbaubarkeit) vorteilhaft sein.

Zur Herstellung von leichtverzweigten Alkylbenzolen (MLAB) wird beispielsweise ein leichtverzweigtes Olefin-Gemisch in Gegenwart eines Alkylierungskatalysators mit Benzol umgesetzt. Die Lage der Phenylgruppe wird dabei durch die Formselektivität des Katalysators bestimmt. Zeolithe des Strukturtyps Mordenit reagieren mit ca. 85 % bevorzugt zu 2-Phenylalkenen, 3-Phenylalkan wird zu ca. 15 % gebildet, siehe Wang et al. Catal. Letters 2001, 76, 1-2.

Tenside mit sehr hohen 2- und 3-Phenylgehalten können den wesentlichen Nachteil aufweisen, dass die Verarbeitbarkeit der Produkte durch einen starken Anstieg der Viskosität der Sulfonate leidet.

Darüber hinaus kann sich ein nicht-optimales Löslichkeitsverhalten ergeben. So ist z.B. der Krafft-Punkt einer Lösung von LAS mit sehr hohen oder sehr niedrigen 2- und 3-Phenylanteilen um bis zu 10-20 °C höher als bei optimaler Wahl des 2- und 3-Phenylanteils.

In der WO 03/029172 sind Verfahren zur Herstellung von Alkylarylverbindungen beschrieben, bei denen aus LPG-, LNG- oder MTO-Strömen stammende C₄-Olefin-Gemische durch Metathese und Dimerisierung in C₁₀₋₁₂-Olefine überführt werden, die sodann zur Alkylierung von aromatischen Kohlenwasserstoffen eingesetzt werden. Durch das Verfahren werden teilverzweigte Alkylarylverbindungen erhalten, die beim Einsatz als Tenside vorteilhafte Eigenschaften aufweisen sollen.

Die DE-A-100 39 995 betrifft Verfahren zur Herstellung von Alkylarylsulfonaten, die durch eine zweistufige Metathese von C₄-Olefinen zu C₁₀₋₁₂-Olefinen, nachfolgend Alkylierung von aromatischen Verbindungen damit und abschließende Sulfonierung und Neutralisation erhalten werden. Als Quellen für die C₄-Olefine werden Crack-Prozesse wie Steam-Cracking oder FCC-Cracking oder die Dehydrierung von Butanen oder die Dimerisierung von Ethen aufgeführt. In den letztgenannten Verfahren können Diene, Alkine oder Enine vor der Metathese mit gängigen Methoden wie Extraktion oder Selektivhydrierung entfernt werden.

Die DE-A-100 59 398 betrifft ebenfalls ein Verfahren zur Herstellung von Alkyarylsulfonaten, wobei im statistischen Mittel vorwiegend einfach verzweigte C₁₀₋₁₄-Olefine mit einem aromatischen Kohlenwasserstoff in Gegenwart eines Alkylierungskatalysators, der Zeolithe des Typs Faujasit enthält, umgesetzt werden. Die C₁₀₋₁₄-Olefine können durch Methathese, Extraktion, Fischer-Tropsch-Synthese, Dimerisierung oder Isomerisierung erhalten werden.

Die bislang zur Alkylierung eingesetzten Olefine weisen teilweise einen zu hohen oder zu niedrigen Verzweigungsgrad auf bzw. ergeben ein nicht optimales Verhältnis terminaler zu interner Phenylalkane.

Aufgabe der vorliegenden Erfindung ist die Bereitstellung eines Verfahrens zur Herstellung von Alkylarylverbindungen und Alkylarylsulfonaten, die zumindest teilweise verzweigt sind und damit für den Einsatz in Wasch- und Reinigungsmitteln gegenüber den bekannten Verbindungen vorteilhafte Eigenschaften aufweisen. Sie sollen insbesondere ein geeignetes Eigenschaftsprofil aus biologischer Abbaubarkeit, Unempfindlichkeit gegen Wasserhärte, Löslichkeit und Viskosität bei der Herstellung und beim Einsatz aufweisen. Zudem sollen die Alkylarylsulfonate kostengünstig herstellbar sein.

Die Aufgabe wird erfindungsgemäß gelöst durch ein Verfahren zur Herstellung von Alkylarylverbindungen durch Umsetzung eines C₁₀-₁₄-Monoolefin-Gemisches mit einem aromatischen Kohlenwasserstoff in Gegenwart eines Alkylierungskatalysators zur Bildung von alkylaromatischen Verbindungen und gegebenenfalls nachfolgende Sulfonierung und Neutralisation der erhaltenen Alkylarylverbindungen, bei denen im Mittel in den C₁₀₋₁₄-Monoolefinen mehr als 0 % und bis zu 100 % Methylverzweigungen in der längsten Kohlenstoffkette vorliegen und weniger als 10 % der Methylverzweigungen in 2-, 3- und 4-Position, berechnet von den Kettenenden der längsten Kohlenstoffkette ausgehend, vorliegen und die C₁₀₋₁₄-Monoolefine jeweils maximal eine Methylverzweigung aufweisen.

Das erfindungsgemäße Verfahren bietet den wesentlichen Vorteil, dass der Einsatz des einzigartigen Olefingemisches nach Alkylierung eines Aromaten, Sulfonierung und Neutralisation ein Tensid liefert, das sich durch eine Kombination von hervorragenden Anwendungseigenschaften (Löslichkeit, Viskosität, Stabilität gegen Wasserhärte, Wascheigenschaften, biologische Abbaubarkeit) auszeichnet. Hinsichtlich der biologischen Abbaubarkeit von Alkylarylsulfonaten sind Verbindungen, die weniger stark an Klärschlamm adsorbiert werden als herkömmliches LAS, besonders vorteilhaft.

Im Alkylierungsschritt kommt es zu einer Gerüstisomerisierung des Olefins wie zum Beispiel Alkylgruppenwanderung. Die Verzweigungsstruktur des eingesetzten OlefinGemisches unterscheidet sich von der in der Seitenkette der entstandenen MLAB-Isomere, d. h. Art und Lage der Verzweigungen können bei der Alkylierung verändert werden. Der Verzweigungsgrad bleibt aber weitestgehend erhalten.

Erfindungsgemäß wurde gefunden, dass zur Herstellung von leichtverzweigten Alkylbenzolen (MLAB) nicht nur der Anteil an einfach methylverzweigtem Olefin und damit der Verzweigungsgrad in der Alkylierung wichtig ist, sondern ebenso die Lage der Methylgruppen. Die weitgehende, vorzugsweise vollständige Abwesenheit von 2-, 3- und 4-Methylverzweigungen im Ausgangsolefin hat sich als besonders günstig für die Produktqualität des MLAB, sowie für die Lebensdauer des Alkylierungskatalysators erwiesen.

Dabei ist es unerheblich, wo sich die Doppelbindung im Olefin befindet, da diese unter den Bedingungen der Alkylierung isomerisiert. Im Sinne dieser Anmeldung und abweichend von den IUPAC-Regeln sollen daher zum Beispiel mit "Methylverzweigung in 2-Position" alle Alkene beschrieben sein, die nach Hydrierung in Alkane übergehen, welche nach den IUPAC-Regeln (Zitat: zum Beispiel *"Hellwinkel"*) zu den 2-Methylalkanen gehören. So ist zum Beispiel 2-Methylundec-2-en mit 10-Methylundec-2-en, aber auch mit zum Beispiel 10-Methylundec-4-en gleichzusetzen. Analoges gilt für die Methylverzweigungen in 3- und 4-Position entlang bzw. in der längsten Kohlenstoffkette. Bei der Umsetzung von Methylalkanen mit Benzol in Gegenwart eines Alkylierungskatalysators kommt es nur in geringem Maße zu einer Gerüstumlagerung. Es werden überwiegend die quartären Alkylbenzole 2-Methyl-2-phenylalkan bzw. 3-Methyl-3-phenylalkan gebildet.

Es ist bekannt, dass sich die Strukturen dieser Art nur schwer biologisch abbauen lassen und daher deren Anwesenheit die Produktqualität vermindert, vergleiche WO 99/05244 und WO 02/092737.

Der Einsatz von 4-Methylalkenen (Alken mit Alkylrest in 4-Position, von einem Ende der längsten Kohlenstoffkette aus betrachtet) in der Alkylierung führt zu einer raschen Deaktivierung des Katalysators. Durch die Bildung von sperrigen 4-Methyl-4-phenyl-alkanen kann es zu einer Blockade der Kanäle oder Belegung der aktiven Zentren des Katalysators kommen.

Erfindungsgemäß wurde gefunden, dass sowohl bei der Umsetzung von 5-Methylalkenen als auch von 6-Methylalkenen (bzw. Alkenen mit Methylresten in 5- oder 6-Position, berechnet von den Kettenenden der längsten Kohlenstoffkette) mit Benzol in Gegenwart des gleichen Alkylierungskatalysators Alkylbenzole mit überwiegend einfach methylverzweigter Seitenkette gebildet werden. Die Lebensdauer des Katalysators ist hoch, der Anteil an quartären MLAB ist gering, obwohl durch Methylgruppenwanderung Isomere entstehen, in denen sich die Methylgruppe in der 2-, 3- oder 4-Position der Seitenkette befindet.

Erfindungsgemäß werden C₁₀₋₁₄-Monoolefin-Gemische eingesetzt. Dabei kann es sich um Gemische von C₁₀-, C₁₁-, C₁₂-, C₁₃- oder C₁₄-Monoolefinen handeln oder auch um Gemische von Monoolefinen mit unterschiedlicher Kettenlänge. Beispielsweise können auch C₁₀-₁₂-Monoolefine mit Kettenlängen in diesem Bereich eingesetzt werden. Üblicherweise handelt es sich bei jeder der Kettenlängen jeweils um Gemische aus verzweigten und unverzweigten Olefinen, bei den verzweigten Olefinen wiederum um unterschiedlich verzweigte Olefine.

Erfindungsgemäß liegen im Mittel in den C₁₀₋₁₄-Monoolefinen mehr als 0 % und bis zu 100 %, vorzugsweise 10 % bis 80 %, besonders bevorzugt 10 % bis 60 % Methylverzweigungen in der längsten Kohlenstoffkette vor, und weniger als 10 %, insbesondere weniger als 5 % der Methylverzweigungen liegen in 2-, 3- und 4-Position vor, berechnet von den Kettenenden der längsten Kohlenstoffkette ausgehend. Die 2-, 3- und 4-Position beziehen sich auf die längste Kohlenstoffkette unabhängig von der Lage der Doppelbindung innerhalb der Kohlenstoffkette. Der Ausdruck "im Mittel" bedeutet, dass beispielsweise bei 100 % Methylverzweigungen statistisch jede Olefinkette eine Methylverzweigung aufweist, wobei in der Praxis im Gemisch Olefine vorliegen können, die keine, eine, zwei oder mehr Methyl-verzweigungen aufweisen. Die C₁₀₋₁₄-Monoolefine weisen jeweils maximal eine Methylverzweigung auf.

Der Anteil und die Position der Methylverzweigungen können durch Gaschromatographie nach den üblichen Verfahren bestimmt werden.

Das Olefingemisch kann aus einer Vielzahl von Quellen stammen und gegebenenfalls durch geeignete Schritte nachbehandelt werden, um das erfindungsgemäße Verzweigungsmuster zu zeigen. Beispielsweise können lineare oder gezielt verzweigte Olefine einem Gemisch zugesetzt werden, oder eine Abtrennung bestimmter Olefine aus dem Gemisch kann durchgeführt werden.

Beispielsweise wird das Olefin erhalten durch
a1) Herstellung eines C₄/C₅-Olefin-Gemisches,
b1) Umsetzung des so erhaltenen C₄/C₅-Olefin-Gemisches an einem Metathesekatalysator zur Herstellung eines 2-Penten und/oder 3-Hexen und/oder 3-Hepten enthaltenden Olefingemisches und gegebenenfalls Abtrennung von 2-Penten und/oder 3-Hexen und/oder 3-Hepten,
c1) Dimerisierung des in Stufe b1) erhaltenen 2-Pentens und/oder 3-Hexens und/oder 3-Heptens an einem Dimerisierungskatalysator zu einem C₁₀₋₁₄-Olefine enthaltenden Gemisch und gegebenenfalls Abtrennung der C₁₀₋₁₄-Olefine.

In Stufe a1) können die C₄-Olefine des C₄/C₅-Olefin-Gemisches, dabei durch Dehydrierung des C₄-Anteils des LPG-, LNG- oder MTO-Stromes und nachfolgende Entfernung von gegebenenfalls gebildeten Dienen, Alkinen und Eninen erhalten werden, wobei der C₄-Anteil des LPG-, LNG- oder MTO-Stromes vor oder nach der Dehydrierung und Entfernung von Dienen, Alkinen und Eninen vom LPG-, LNG- oder MTO-Strom abgetrennt wird. Der LNG-Strom kann über ein MTO-Verfahren in das C₄-Olefingemisch überführt werden.

In Stufe c1) können auch Heptene beigemischt werden.

Das Olefingemisch kann auch erhalten werden durch
a2) Herstellung eines C₅₋₇-Olefin-Gemisches durch Dehydrierung von C₅₋₇-Alkanen mit gegebenenfalls vor- oder nachgeschalteter Isomerisierung,
b2) Dimerisierung des in Stufe a2) erhaltenen C₅₋₇-Olefingemisches an einem Dimerisierungskatalysator zu einem C₁₀₋₁₄-Olefine enthaltenden Gemisch und gegebenenfalls Abtrennung der C₁₀₋₁₄-Olefine.

Geeignete Aromaten sind Benzol, Toluol, Ethylbenzol und die Xylole, bevorzugt sind Benzol, Toluol und Ethylbenzol, besonders bevorzugt ist Benzol.

Geeignete Katalysatoren sind Zeolithe der Strukturtypen EPI, FER, Pentasile mit MFI- oder MEL-Struktur, Faujasite wie z. B. Y, LTL, MOR, BEA, GME, MAZ. Bevorzugt sind LTL, FAU inklusive den USY-Typen, BEA und MOR. Besonders bevorzugt ist MOR.

Diese Zeolithe werden bevorzugt in der H- u/o A1-Form eingesetzt, jedoch können je nach Herstellung noch Spuren an Na, K, Mg oder Ca anwesend sein. Ein teilweiser oder vollständiger Austausch des Gitter-Aluminiums durch B, Ga oder Fe ist möglich.

Der Katalysator kann als feines Pulver in Suspension direkt verwendet werden, bei Zeolithen sind dies z. B. Teilchengrößen zwischen 100 nm und einigen µm. Meist werden diese Katalysatoren jedoch zusammen mit Bindermaterialien zu Formkörpers von 0,1 - 5 mm Durchmesser verformt. Zum Einsatz in Festbetten sind 1 - 3 mm bevorzugt, in Suspension 0,001 - 1 mm, in Fließbetten 0,1 - 3 mm. Als Binder eignen sich besonders Tone, Aluminiumoxide wie z. B. Purale, Sirale und Versale und Kieselgele. Weiter können inerte Füllstoffe wie SiO₂ (z. B. Aerosil von Degussa) zugesetzt werden.

Als Formkörper eignen sich Tabletten, Stränglinge, Ringe, Rippstränge, Stern- oder Wagenradextrudate.

Die Katalysatoren besitzen vorzugsweise spezifische Oberflächen von 30 bis 2000 m²/g, bevorzugt 100 bis 700 m²/g. Das Volumen der Poren mit Durchmesser 2 - 20 nm beträgt typischerweise 0,05 - 0,5 ml/g, bevorzugt 0,01 bis 0,1 ml/g, das der Poren von 200 - 2000 nm typischerweise 0,05 - 0,5 ml/g, bevorzugt 0,05 bis 0,3 ml/g.

Deaktivierte Katalysatoren können in den meisten Fällen zum Beispiel durch Abbrennen in Luft oder Magerluft bei 250 - 550 °C reaktiviert werden. Alternativ ist eine Behandlung mit bei tieferer Temperatur - optional auch in der Flüssigphase - oxidierend wirkenden Verbindungen möglich, hier sind insbesondere, NOₓ, H₂O₂ und die Halogene zu nennen. Die Regenerierung kann direkt im Alkylierungsreaktor oder extern erfolgen.

Die Alkylierung findet bevorzugt in der Flüssigphase, d. h. ohne Gasphase statt, was durch einen entsprechenden Systemdruck erreicht werden kann. Alkylierungstemperaturen sind bevorzugt 100 bis 250 °C, besonders bevorzugt 120 bis 220 °C, ganz besonders bevorzugt 130 bis 220 °C, speziell 150 °C bis 180 °C, z. B. 160 °C.

Geeignete Reaktoren sind z. B. alle Systeme mit Rührkesselcharakteristik, d. h. Rührkessel, Schlaufenreaktoren, Reaktoren mit externem Umlauf, Strahlschlaufenreaktioren sowie Fließ- oder Wanderbetten.

Die Erfindung betrifft auch Alkylarylverbindungen, insbesondere Alkylbenzolsulfonate, die nach dem erfindungsgemäßen Verfahren erhältlich sind. Die Alkylbenzolsulfonate können vorzugsweise als Tenside, insbesondere in Wasch- und Reinigungsmitteln eingesetzt werden.

Die Erfindung betrifft auch Wasch- und Reinigungsmittel, die die erfindungsgemäßen Alkylbenzolsulfonate neben üblichen Inhaltsstoffen enthalten. Für übliche Inhaltsstoffe und Waschmittelzusammensetzungen kann auf die WO 03/029172 verwiesen werden. Dort sind übliche Inhaltsstoffe wie Bleichmittel, Bleichaktivatoren, Bleichstabilisatoren, anorganische Builder (Gerüstsubstanzen), anionische Tenside, nichtionische Tenside, organische Cobuilder, Vergrauungsinhibitoren und Soi-Release-Polymere, Farbübertragungsinhibitoren, Enzyme usw. aufgeführt.

Ein typisches erfindungsgemäßes pulver- oder granulatförmiges Vollwaschmittel kann beispielsweise folgende Zusammensetzung aufweisen:
- 0,5 bis 50 Gew.-%, vorzugsweise 5 bis 30 Gew.-%, mindestens eines anionischen und/- oder nichtionischen Tensids,
- 0,5 bis 60 Gew.-%, vorzugsweise 15 bis 40 Gew.-%, mindestens eines anorganischen Builders,
- 0 bis 20 Gew.-%, vorzugsweise 0,5 bis 8 Gew.-%, mindestens eines organischen Cobuilders,
- 2 bis 35 Gew.-%, vorzugsweise 5 bis 30 Gew.-%, eines anorganischen Bleichmittels,
- 0,1 bis 20 Gew.-%, vorzugsweise 0,5 bis 10 Gew.-%, eines Bleichaktivators, gegebenenfalls in Abmischung mit weiteren Bleichaktivatoren,
- 0 bis 1 Gew.-%, vorzugsweise bis höchstens 0,5 Gew.-%, eines Bleichkatalysators,
- 0 bis 5 Gew.-%, vorzugsweise 0 bis 2,5%, eines polymeren Farbübertragungsinhibitors,
- 0 bis 1,5 Gew.-%, vorzugsweise 0,1 bis 1,0 Gew.-%, Protease,
- 0 bis 1,5 Gew.-%, vorzugsweise 0,1 bis 1,0 Gew.-%, Lipase,
- 0 bis 1,5 Gew.-%, vorzugsweise 0,2 bis 1,0 Gew.-% eines Soil-Release-Polymers, ad 100% übliche Hilfs- und Begleitstoffe und Wasser.

Vorzugsweise in Waschmitteln eingesetzte anorganische Builder sind Natriumcarbonat, Natriumhydrogencarbonat, Zeolith A und P sowie amorphe und kristalline Na-Silikate.

Vorzugsweise in Waschmitteln eingesetzte organische Cobuilder sind Acrylsäure/MaleinsäureCopolymere, Acrylsäure/Maleinsäure/Vinylester- Terpolymere und Citronensäure.

Vorzugsweise in Waschmitteln eingesetzte anorganische Bleichmittel sind Natriumperborat und Natriumcarbonat-Perhydrat.

Vorzugsweise in Waschmitteln eingesetzte anionische Tenside sind die erfindungsgemäßen linearen und leicht verzweigten Alkylbenzolsulfonate (LAS), Fettalkoholsulfate und Seifen.

Vorzugsweise in Waschmitteln eingesetzte nichtionische Tenside sind C₁₁- bis C₁₇-Oxoalkoholethoxylate mit 3-13 Ethylenoxid-Einheiten, C₁₀- bis C₁₆-Fettalkoholethoxylate mit 3-13 Ethylenoxideinheiten sowie zusätzlich mit 1-4 Propylenoxid- oder Butylenoxid-Einheiten alkoxylierte ethoxylierte Fett- oder Oxoalkohole.

Vorzugsweise in Waschmitteln eingesetzte Enzyme sind Protease, Lipase und Cellulase. Von den handelsüblichen Enzymen werden dem Waschmittel in der Regel Mengen von 0,05 bis 2,0 Gew.-%, vorzugsweise 0,2 bis 1,5 Gew.-%, des konfektionierten Enzyms zugesetzt. Geeignete Proteasen sind z.B Savinase, Desazym und Esperase (Hersteller Novo Nordisk). Eine geeignete Lipasen ist z.B. Lipolase (Hersteller Novo Nordisk). Eine geeignete Cellulase ist z.B. Celluzym (Hersteller Novo Nordisk).

Vorzugsweise in Waschmitteln eingesetzte Vergrauungsinhibitoren und Soil-Release-Polymere sind Pfropfpolymere von Vinylacetat auf Polyethylenoxid der Molmasse 2.500-8.000 im Gewichtsverhältnis 1,2:1 bis 3,0:1, Polyethylenterephthalate/Oxyethylenterephthalate der Molmasse 3.000 bis 25.000 aus Polyethylenoxiden der Molmasse 750 bis 5.000 mit Terephthalsäure und Ethylenoxid und einem Molverhältnis von Polyethylenterephthalat zu Polyoxyethylenterephthalat von 8:1 bis 1:1 sowie Blockpolykondensate gemäß DE-A-44 03 866.

Vorzugsweise in Waschmitteln eingesetzte Farbübertragungsinhibitoren sind lösliche Vinylpyrrolidon- und Vinylimidazol-Copolymere mit Molmassen über 25.000 sowie feinteilige vernetzte Polymere auf Basis Vinylimidazol.

Die erfindungsgemäßen pulver- oder granulatförmigen Waschmittel können bis zu 60 Gew.-% anorganischer Stellmittel enthalten. Üblicherweise wird hierfür Natriumsulfat verwendet. Vorzugsweise sind die erfindungsgemäßen Waschmittel aber arm an Stellmitteln und enthalten nur bis zu 20 Gew.-%, besonders bevorzugt nur bis 8Gew.-% an Stellmitteln.

Die erfindungsgemäßen Waschmittel können unterschiedliche Schüttdichten im Bereich von 300 bis 1.200, insbesondere 500 bis 950g/l, besitzen. Moderne Kompaktwaschmittel besitzen in der Regel hohe Schüttdichten und zeigen einen Granulataufbau.

Die Erfindung wird anhand der nachstehenden Beispiele näher erläutert.

### Beispiele

### Beispiel 1:

Ein in einem Umluftofen befindlicher Rohrreaktor wurde mit 32 g Katalysatorsplitt (60 % H-Mordenit mit SiO₂:Al₂O₃ = 24,5 - verformt mit 40 % Pural SB von Condea) der Korngröße 0,7 - 1,0 mm gefüllt und 6 h bei 500 °C aktiviert. Dann wurde abgekühlt, mit einem Zulauf aus Benzol: 5-Methylundecen (4:1 molar) geflutet, mit 0.44 g/g_{Kat}h belastet und ein zehnfach höherer Umlaufstrom eingestellt. Schließlich wurde der Reaktor auf 160 °C (einphasig flüssig, 30 bar hydraulischer Druck) erhitzt, und zeitaufgelöst wurde der Gehalt an Edukten und Produkten im Auslaufstrom mittels GC detektiert. Das erhaltene C₁₈-Alkylarylgemisch wurde destillativ gereinigt und mittels Gaschromatographie-Massenspektrometrie Kopplung und ¹H/¹³C-NMR analysiert. Nach 86 Stunden wurde ein Rückgang des Dodecen-Umsatzes auf unter 95 % beobachtet.

### Beispiel 2 (Vergleich):

In der Apparatur aus Beispiel 1 wurde unter analogen Bedingungen ein Zulauf aus Benzol: 4-Methylundecen (4:1 molar) umgesetzt. Der Rückgang des Dodecen-Umsatzes unter 95 % wurde bereits nach 30 Stunden beobachtet.

### Beispiel 3:

In einem Autoklaven wurden unterschiedliche Methylundecene, wie nachstehend angegeben, mit Benzol im molaren Verhältnis 1:10 an einem Mordenit-Katalysator (5 Gew.-%) bei verschiedenen Temperaturen umgesetzt. Die Ergebnisse sind in den nachstehenden Tabellen zusammengefasst:

**Umsetzung von 2-Methylundecen (Vergleich):**

| **Reaktionstemperatur** | **Reaktionsprodukte** | |
|---|---|---|
| | **2-m-2-p** | **3-m-3-p** |
| 100 °C | 100% | <1% |
| 120 °C | 94.9% | <1% |
| 140 °C | 88.7 % | <1% |
| 160 °C | 86.9 % | <1% |
| 180 °C | 77.8 % | <1% |
| 200 °C | 42.5 % | <1% |

**Umsetzung von 3-Methylundecen (Vergleich):**

| **Reaktionstemperatur** | **Reaktionsprodukte** | | |
|---|---|---|---|
| | **2-m-2-p** | **3-m-2-p** | **3-m-3-p** |
| 100°C | 13.6 % | 47.4 % | 15.6 % |
| 120 °C | 16.1 % | 50.4 % | 12.7 % |
| 140°C | 14.4 % | 46.6 % | 8.9 % |
| 160 °C | 13.8 % | 44.0 % | 6.3 % |
| 180 °C | 19.3 % | 39.8 % | 8.0 % |
| 200 °C | 21.9 % | 27.4 % | 4.7 % |

**Umsetzung von 5-Methylundecen:**

| **Reaktionstemperatur** | **Reaktionsprodukte** | | |
|---|---|---|---|
| | **5/7-m-2-p** | **2-m-2-p** | **3-m-3-p** |
| 100°C | 47.9 % | 1.6 % | <1 % |
| 120 °C | 50.0 % | 2.2 % | <1 % |
| 140°C | 47.6 % | 3.2 % | <1 % |
| 160 °C | 47.5 % | 2.1 % | <1 % |
| 180 °C | 30.3 % | 2.9 % | <1 % |
| 200 °C | 23.3 % | 2.0 % | <1 % |

**Umsetzung von 6-Methylundecen:**

| **Reaktionstemperatur** | **Reaktionsprodukte** | | |
|---|---|---|---|
| | **6-m-2-p** | **2-m-2-p** | **3-m-3-p** |
| 160 °C | 48.0 | 1.8 % | <1 % |
| 180 °C | 40.7 | 3.2 % | <1 % |

In den Abkürzungen für die Produkte bedeuten m Methyl und p Phenylundecan. 2-m-2-p bedeutet somit 2-Methyl-2-phenylundecan.

### Beispiel 4 (Vergleich):

In WO 01/05733 A1 (UOP) wird die Zusammensetzung eines Olefins *ex* Isomerisierung/Dehydrierung wie folgt angegeben:

| **Olefin** | **Gehalt** |
|---|---|
| Leichte Olefine | 0.64 % |
| Lineare Olefine | 30.11 % |
| 6-Methylundecen | 7.66 % |
| 5-Methylundecen | 15.33 % |
| 4-Methylundecen | 11.82 % |
| 3-Methylundecen | 12.95 % |
| 2-Methylundecen | 8.87 % |
| andere verzweigte Olefine | 9.05 % |
| Höhere Isomere | 3.53 % |
| Summe | 99.96 % |

Eine Mischung dieses Olefins mit Benzol (93.3 % Benzol, 6.7 % Olefin, molares Verhältnis Benzol: Olefin ca. 30:1) wurde in Gegenwart von 75 mL (53.0 g) Mordenit mit einer LHSV von 2.0 h-1 (Belastung von 0.15 g Olefin/g Kat) bei 125 °C und 35 bar umgesetzt.

Die 2-Phenylselektivität im Produkt wurde mit 82.0 %, der Anteil an 2-Methyl-2-Phenylundecan mit 6.98 % und der Anteil an höheren Quarts mit 1.9 % bestimmt.

### Beispiel 5:

Unter den oben in Beispiel 4 angegebenen Bedingungen wurde ein Olefin aus der Kette Olefin-Hydroformylierung-Aldokendensation-Hydrierung-Dehydratisierung oder gemäß Beispiel 3 der WO 03/029172 eingesetzt.

| **Olefin** | **Gehalt** |
|---|---|
| Lineare Olefine | 14.0 % |
| 5-Methylundecen | 35.0 % |
| 4-Ethyldecen | 32.5 % |
| Höher verzweigte Olefine | 17.0 % |
| Höhere Isomere | 1.4 % |
| Summe | 99.9 % |

Die 2-Phenylselektivität im Produkt wurde mit 82.4 %, der Anteil an 2-Methyl-2-Phenylundecan mit 2.33 % und der Anteil an höheren Quarts mit 0.8 % bestimmt.

## Patentansprüche

1. Verfahren zur Herstellung von Alkylarylverbindungen durch Umsetzung eines C₁₀₋₁₄-Monoolefin-Gemisches mit einem aromatischen Kohlenwasserstoff in Gegenwart eines Alkylierungskatalysators zur Bildung von alkylaromatischen Verbindungen und gegebenenfalls nachfolgende Sulfonierung und Neutralisation der erhaltenen Alkylarylverbindungen, **dadurch gekennzeichnet, dass** im Mittel in den C₁₀₋₁₄-Monoolefinen mehr als 0 % und bis zu 100 % Methylverzweigungen in der längsten Kohlenstoffkette vorliegen und weniger als 10 % der Methylverzweigungen in 2-, 3- und 4-Position, berechnet von den Kettenenden der längsten Kohlenstoffkette ausgehend, vorliegen, und die C₁₀₋₁₄-Monoolefine jeweils maximal eine Methylverzweigung aufweisen.

2. Verfahren nach Anspruch 1, **dadurch gekennzeichnet, dass** im Mittel in den C₁₀₋₁₄-Monoolefinen 10 bis 80 % Methylverzweigungen in der längsten Kohlenstoffkette vorliegen.

3. Verfahren nach Anspruch 1 oder 2, **dadurch gekennzeichnet, dass** der aromatische Kohlenwasserstoff Benzol ist.

4. Verfahren nach einem der Ansprüche 1 bis 3, **dadurch gekennzeichnet, dass** der Alkylierungskatalysator ausgewählt ist aus Zeolithen der Strukturtypen EPI, FER, Pentasilen mit MFI- oder MEL-Struktur oder Faujasiten.

5. Verfahren nach einem der Ansprüche 1 bis 4, **dadurch gekennzeichnet, dass** die Alkylierung in der Flüssigphase bei einer Temperatur im Bereich von 100 bis 250 °C durchgeführt wird.

6. Alkylarylverbindungen, erhältlich nach dem Verfahren gemäß einem der Ansprüche 1 bis 5.

7. Verwendung von Alkylarylsulfonaten gemäß Anspruch 6 als Tenside.

8. Wasch- oder Reinigungsmittel enthaltend neben üblichen Inhaltsstoffen Alkylarylsulfonate gemäß Anspruch 6.

## Claims

1. A process for preparing alkylaryl compounds by reacting a C₁₀₋₁₄-monoolef in mixture with an aromatic hydrocarbon in the presence of an alkylation catalyst to form alkyl aromatic compounds and optionally subsequently sulfonating and neutralizing the resulting alkylaryl compounds, wherein, in the C₁₀₋₁₄-monoolefins, on average, more than 0% and up to 100% of methyl branches are present in the longest carbon chain and fewer than 10% of the methyl branches are in the 2-, 3- and 4-position, calculated starting from the chain ends of the longest carbon chain, and the C₁₀₋₁₄-monoolefins in each case have a maximum of one methyl branch.

2. The process according to claim 1, wherein, in the C₁₀₋₁₄-monoolefins, on average, from 10 to 80% of methyl branches are present in the longest hydrocarbon chain.

3. The process according to claim 1 or 2, wherein the aromatic hydrocarbon is benzene.

4. The process according to any of claims 1 to 3, wherein the alkylation catalyst is selected from zeolites of the EPI, FER structural types, pentasils having MFI or MEL structure and faujasites.

5. The process according to any of claims 1 to 4, wherein the alkylation is carried out in the liquid phase at a temperature in the range from 100 to 250°C.

6. An alkylaryl compound obtainable by the process according to any of claims 1 to 5.

7. The use of alkylarylsulfonates according to claim 6 as surfactants.

8. A laundry detergent or cleaning composition comprising, in addition to customary ingredients, alkylarylsulfonates according to claim 6.

## Revendications

1. Procédé de fabrication de composés d'alkylaryle par mise en réaction d'un mélange de monooléfines en C₁₀₋₁₄ avec un hydrocarbures aromatique en présence d'un catalyseur d'alkylation pour former des composés alkylaromatiques, puis éventuellement sulfonation et neutralisation des composés d'alkylaryle obtenus, **caractérisé en ce qu'**en moyenne, plus de 0 % et jusqu'à 100 % de ramifications méthyle sont présentes dans la chaîne carbonée la plus longue dans les monooléfines en C₁₀₋₁₄, et moins de 10 % des ramifications méthyle sont présentes en position 2, 3 et 4, en partant des extrémités de la chaîne carbonée la plus longue, et les monooléfines en C₁₀₋₁₄ comprennent chacune au plus une ramification méthyle.

2. Procédé selon la revendication 1, **caractérisé en ce qu'**en moyenne, 10 à 80 % de ramifications méthyle sont présentes dans la chaîne carbonée la plus longue dans les monooléfines en C₁₀₋₁₄.

3. Procédé selon la revendication 1 ou 2, **caractérisé en ce que** l'hydrocarbure aromatique est le benzène.

4. Procédé selon l'une quelconque des revendications 1 à 3, **caractérisé en ce que** le catalyseur d'alkylation est choisi parmi les zéolithes des types structuraux EPI, FER, les pentasiles de structure MFI ou MEL, ou les faujasites.

5. Procédé selon l'une quelconque des revendications 1 à 4, **caractérisé en ce que** l'alkylation est réalisée dans la phase liquide à une température dans la plage allant de 100 à 250 °C.

6. Composés d'alkylaryle, pouvant être obtenus par le procédé selon l'une quelconque des revendications 1 à 5.

7. Utilisation de sulfonates d'alkylaryle selon la revendication 6 en tant que tensioactifs.

8. Détergent contenant en plus des composants usuels des sulfonates d'alkylaryle selon la revendication 6.
